(19) Europäisches Patentamt
European Patent Office
Office européen des brevets

(11) Numéro de publication: **0 101 381**
**B1**

(12) # FASCICULE DE BREVET EUROPEEN

(45) Date de publication du fascicule du brevet: **04.12.85**

(51) Int. Cl.⁴: **C 07 D 401/06**, C 07 D 211/70, A 61 K 31/445

(21) Numéro de dépôt: **83401639.6**

(22) Date de dépôt: **10.08.83**

(54) **Trifluorométhylphényltétrahydropyridines à activité anorexigène, un procédé de préparation et compositions pharmaceutiques.**

(30) Priorité: **16.08.82 FR 8214169**

(43) Date de publication de la demande:
**22.02.84 Bulletin 84/8**

(45) Mention de la délivrance du brevet:
**04.12.85 Bulletin 85/49**

(84) Etats contractants désignés:
**AT BE CH DE FR GB IT LI LU NL SE**

(56) Documents cités:
**EP - A - 0 060 176**
**EP - A - 0 060 179**
**DE - A - 2 101 997**
**FR - A - 2 416 886**
**FR - E - 86 403**
**GB - A - 948 071**

(73) Titulaire: **SANOFI, société anonyme, 40, Avenue George V, F-75008 Paris (FR)**

(84) Etats contractants désignés: **AT BE CH DE FR GB IT LI LU NL SE**

(73) Titulaire: **MIDY S.p.A., Société Anonyme dite:, Via Piranesi 38, I-20137 Milano (IT)**

(84) Etats contractants désignés: **IT**

(72) Inventeur: **Nisato, Dino, Viale Golgi, 80, Pavia (IT)**
Inventeur: **Frigerio, Marco, Via Carlo Poma, 28, Mantova (IT)**
Inventeur: **Miranda, Giovana F., Via Oderzo, 4, Milano (IT)**

(74) Mandataire: **Gillard, Marie-Louise et al, Cabinet Beau de Loménie 55, Rue d'Amsterdam, F-75008 Paris (FR)**

EP 0 101 381 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux composés à activité anorexigène.

Plus particulièrement, l'invention se réfère à de nouvelles 4-(3-trifluorométhylphényl)-1,2,3,6-tétrahydropyridines de formule

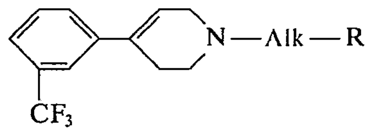

(I)

dans laquelle R représente un groupe pyridyle, un groupe pyridyle l-oxyde ou un groupe naphtyle, non substitués ou substitués par un groupe alkyle contenant de 1 à 4 atomes de carbone et Alk représente un groupe alkylène à chaine droite ou ramifiée contenant de 2 à 4 atomes de carbone, ainsi qu'à leurs sels pharmaceutiquement acceptables, ayant un'activité anorexigène remarquable.

Les produits de formule I dans laquelle R est pyridyle 1-oxyde sont particulièrement préférés.

Il est connu que le chef de file des composés à action anorexigène est l'amphétamine qui exerce son activité par un mécanisme d'action biochimique central au niveau des systèmes dopaminergique et noradrénergique.

L'amphétamine et ses dérivés présentent des inconvénients importants car leur effet stimulant le système nerveux central ainsi que la possibilité d'accoutumance et de pharmacodépendance peuvent constituer un danger potentiel pour le patient.

Des études ont été donc consacrées à la recherche de dérivés de l'amphétamine présentant une dissociation entre l'effet stimulant et l'action anorexigène. L'introduction d'un groupe trifluorométhyle dans la position »méta« du groupe phényle de l'éthylamphétamine a donné lieu à l'obtention àun produit, doté d'une excellente activité anorexigène, ci-après désigné par sa Dénomination Commune Internationale »»fenfluramine«, qui, au lieu d'être stimulant, possède une certaine action sédative.

L'avantage de la fenfluramine et de ses dérivés par rapport à l'amphétamine et à ses dérivés est dû à la différence de mécanisme d'action. En fait, l'anorexie provoquée par l'amthétamine semble être médiée par la libération de noradrénaline cérébrale, alors que l'action anorexigène de la fenfluramine dépend de la libération de la sérotonine endogène des neurones centraux (Ann. C. Sullivan et al., Appetite Regulation and Its Modulation by Drugs. Nutrition and Drug Interrelation, 1978, Academic Press, 21—28) et de l'inhibition du captage de la sérotonine.

Il est cependant connu que la fenfluramine, à des doses très proches à la dose anorexigène, provoque une réduction significative des taux cérébraux de sérotonine (Arch. Intern. Pharmacodyn. Ther. 1967, 170, 276) et qu'une déplétion durable de sérotonine peut être considérée comme un signe de neurotoxicité potentielle (C. D. Morgan et al., Life Sci. 1972, Part I, 11, 83).

Le brevet français 2 416 886 décrit quant á lui des dérivés de 3-(3-trifluorométhyl-phényl)-tetrahydropyridine, N-substitués par un phenyl-alcoyle et possidant des propriétés anorexigène.

On a maintenant trouvé que les 4-(3-trifluorométhyl-phényl)1,2,3,6-tétrahydropyridines de formule I ci-dessus, et leurs sels, montrent une activité anorexigène remarquable associée à une toxicité très faible. Sur le plan biochimique, les composés de formule I ci-dessus, ainsi que leurs sels, agissent avec un mécanisme d'action différent de celui de l'amphétamine et de la fenfluramine car ils ne provoquent aucune stimulation du système nerveux central ni de libération de sérotonine neuronale. Plus particulièrement, les composés de formule I ci-dessus montrent une grande affinité pour les récepteurs post-synaptiques de la sérotonine comportant une activité anorexigène sans les effets secondaires dûs à la déplétion de sérotonine.

Ainsi, la présente invention a pour objet les nouvelles 4-(3-trifluorométhylphényl)-1,2,3,6-tétrahydropyridines de formule I ci-dessus, ainsi que leurs sels pharmaceutiquement acceptables.

Les sels pharmaceutiquement acceptables comprennent les sels non toxiques dérivés d'acides minéraux ou organiques tels que le chlorhydrate, le bromhydrate, le succinate, le tartrate, le citrate, le fumarate, le maléate, le 4,4'-méthylènebis-(3-hydroxy-2-naphtoate), le 2-naphtalènesulfonate, le méthanesulfonate, le p-toluènesulfonate et similaires.

Selon un autre de ses aspects, la présente invention se réfère à procédé pour la préparation des 4-(3-trifluorométhylphényl)-1,2,3,6-tétrahydropyridines de formule I ci-dessus et de leurs sels.

Ledit procédé est caractérisé en ce qu'on fait réagir, dans un solvant organique et à une température entre 20 et 200°C, la 4-(3-trifluorométhylphényl)-1,2,3,6-tétrahydropyridine de formule

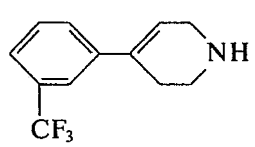

(II)

avec un composé de formule X—R, ou R a la signification donnée ci-dessus et X représente un groupe chloro-, bromo- ou iodoalkyle ayant de 1 à 4 atomes de carbone ou un groupe électrophile, tel que le

groupe méthanesulfonyloxy ou p-toluènesulfonyloxy, ou bien, lorsque R est autre que naphtyle éventuellement substitué, un groupe vinyle non substitué ou substitué par 1 ou 2 groupes méthyle ou par un groupe éthyle.

La 4-(3-trifluorométhylphényl)-1,2,3,6-tétrahydropyridine utilisée comme produit de départ est bien connue dans la littérature.

Comme solvant organique préférentiel, on utilise un alcool aliphatique ayant de 1 à 6 atomes de carbone, tel que méthanol, éthanol, n-butanol, n-pentanol, mais d'autres solvants tels que hexane, diméthylformamide, diméthylsulfoxyde, sulfolane, acétonitrile, pyridine et similaires peuvent être utilisés.

La réaction est avantageusement conduite en présence d'un agent de condensation basique, tel que la triéthylamine, surtout dans le cas où le réactif R–X est un dérivé halogéné.

La température de réaction peut varier entre la température ambiante (environ 20°C) et 200°C et les temps de réaction varient de conséquence. En général, après 4 à 5 h de chauffage à 100–150°C, la réaction est complète et le produit final ainsi abtenu peut être isolé selon les techniques conventionnelles et éventuellement transformé dans ses sels par simple salification dans un solvant organique tel qu'un alcool, éthanol ou isopropanol de préférence, un éther comme le 1,2-diméthoxyéthane, l'acétate d'éthyle ou un hydrocarbure comme l'hexane.

Selon un autre de ses aspects, la présente invention concerne un procédé pour la préparation des composés de formule I ci-dessus dans laquelle Alk est à chaîne droite, à savoir des composés de formule

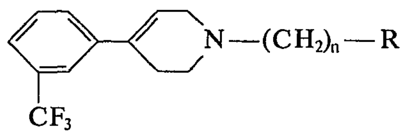

(III)

où R est tel que défini ci-dessus et n est 1, 2, 3 ou 4, caractérisé en ce qu'on réduit un composé de formule

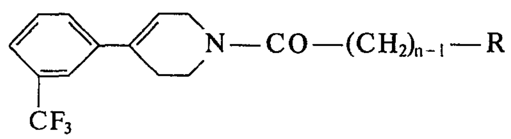

(IV)

dans laquelle R et n sont tels que définis ci-dessus, par un hydrure d'aluminium ou par un hydrure complexe de lithium et d'aluminium dans un solvant organique inerte à une température entre 0°C et la température d'ébullition du solvant employé et l'on transforme éventuellement le produit ainsi obtenu dans ses sels pharmaceutiquement acceptables.

La réaction de réduction est conduite selon les modes opérationnels connus en soi en utilisant comme agent de réduction l'hydrure d'aluminium ou un hydrure complexe de lithium et d'aluminium, tel que $LiAlH_4$, $LiAlh(OCH_3)_3$ et similaires. On opère en général dans un solvant inerte comme un éther, tel que l'éther diéthylique, le tétrahydrofuranne, le dioxane ou le 1,2-diméthoxyéthane.

Selon un mode opérationnel préférentiel, on opère avec une quantité équimoléculaire d'hydrure de lithium et d'aluminium $LiAlH_4$ par rapport au composé IV de départ, à une température de 20–30°C dans de l'éther diéthylique et sous atmosphère inerte. Après environ 1 h, la réduction est complète et le composé de formule III est isolé selon les techniques conventionnelles sous forme de base libre ou d'un de ses sels.

La base libre peut être transformée dans un de ses sels par simple salification dans un solvant organique tel qu'un alcool, éthanol ou isopropanol de préférence, un éther comme le 1,2-diméthoxyéthane, l'acétate d'éthyle ou un hydrocarbure comme l'hexane.

Les composés de formule IV ci-dessus sont préparés en faisant réagir la 4-(3-trifluorométhylphényl)-1,2,3,6-tétrahydropyridine de formule II avec un dérivé fonctionnel d'un acide carboxylique de formule

$$R-(CH_2)_{n-1}-COOH \qquad (V)$$

où R et n sont tels que définis ci-dessus, dans un solvant organique à une température comprise entre −10°C et la température d'ébullition du solvant employé.

Comme dérivé fonctionnel approprié, on peut utiliser l'acide libre activé, l'anhydride, un anhydride mixte, un ester actif ou un halogénure d'acide, le chlorure de préférence. Parmi les esters actifs, l'ester de p-nitrophényle est particulièrement préféré, mais les esters de méthoxyphényle, de trityle, de benzhydryle et similaires sont également convenables.

La température de réaction peut varier entre −10°C et la température d'ébullition du solvant employé, mais en général on opère à la température ambiante ou à 30–50°C. Il peut être préférable de conduire la réaction au froid lorsqu'elle est exothermique, comme dans le cas où on utilise le chlorure en tant que dérivé fonctionnel de l'acide de formule V.

Comme solvant de réaction, on utilise de préférence un alcool, tel que le méthanol ou l'éthanol, ou un solvant halogéné tel que le chlorure de méthylène, le dichloroéthane, le chloroforme et similaires, mais d'autres solvants organiques compatibles avec les réactifs employés, par exemple le dioxane, le tétrahydrofuranne ou un hydrocarbure tel que l'hexane peuvent être également employés.

La réaction peut être conduite en présence d'un accepteur de protons, par exemple d'un carbonate alcalin ou d'une amine tertiaire, dans le cas où de l'acide chlorhydrique, ou un autre acide, se libérerait pendant la réaction, mais cet accepteur de protons n'est pas indispensable pour l'obtention du produit final.

Le produit que l'on obtient à la fin de la réaction est en général une huile qui peut être isolée et caractérisée selon les techniques conventionnelles, mais qui peut être utilisée à l'état brut pour la réduction avec l'hydrure.

Les 4-(3-trifluorométhylphényl)-1,2,3,6-tétrahydropyridines de la présente invention et leurs sels montrent une activité anorexigène remarquable et sélective sans donner aucun effet du type amphétaminique. La sélectivité de leur action anorexigène est démontrée par l'absence d'activités pharmacologiques secondaires, telles que l'activité sédative ou excitante et l'action inhibitrice de l'activité locomotrice.

L'activité anorexigène a été évaluée par la méthode de la prise de nourriture chez le rat. On a utilisé des rats femelles de 200 g entrainés pendant 10 jours à manger durant une période de 4 h et sélectionnés au huitième jour. Au bout du dixième jour, les animaux randomisés ont été divisés en un »groupe contrôle«, traité par le seul véhicule, et en plusieurs »groupes traités«. Le traitement a été effectué par voie intrapéritonéale ou orale respectivement 30 min ou 1 h avant la présentation de la nourriture et, ensuite, on a mesuré la quantité de nourriture consommée durant la première heure.

Le tableau I montre, pour sept composés représentatifs de l'invention:

- la toxicité aiguë, exprimée comme DL 50 chez le rat par voie orale ou par voie intrapéritonéale (donnée A);
- l'activité anorexigène, exprimée comme dose orale ou intrapéritonéale inhibant de 50% la prise de nourriture (DI 50, donnée B);
- le rapport entre la toxicité aiguë et l'activité anorexigène, qui exprime l'indice thérapeutique lié à la toxicité aiguë (donnée A/B).

Comme produits représentatifs de la présente invention, on a utilisé les composés suivants:

- le chlorhydrate de 1-[2-(2-pyridyl)éthyl]-4-(3-trifluorométhylphényl)-1,2,3,6-tétrahydropyridine (composé de l'Exemple 1),
- le chlorhydrate de 1-[2-(3-pyridyl)éthyl]-4-(3-trifluorométhylphényl)-1,2,3,6-tétrahydropyridine (composé de l'Exemple 2),
- le chlorhydrate de 1-[2-(4-pyridyl)éthyl]-4-(3-trifluorométhylphényl)-1,2,3,6-tétrahydropyridine (composé de l'Exemple 3),
- le chlorhydrate de 1-[2-(6-méthyl-2-pyridyl)éthyl]-4-(3-trifluorométhylphényl)-1,2,3,6-tétrahydropyridine (composé de l'Exemple 4),
- le chlorhydrate de 4-[2-[4-(3-trifluorométhylphényl)-1,2,3,6-tétrahydropyrid-1-yl]éthyl]pyridine 1-oxyde (composé de l'Exemple 5),
- le chlorhydrate de 1-[2-(1-naphtyl)éthyl]-4-(3-trifluorométhylphényl)-1,2,3,6-tétrahydropyridine (composé de l'Exemple 6),
- le chlorhydrate de 1-[2-(2-naphtyl)éthyl]-4-(3-trifluorométhylphényl)-1,2,3,6-tétrahydropyridine (composé de l'Exemple 7).

Tableau I

| Composé | Voie d'administration | A<br>DL50<br>mg/kg | B<br>DI50<br>mg/kg | A/P |
|---|---|---|---|---|
| Exemple 1 | i. p. | 107,8 | 1,99 | 54,2 |
| | os | 195,3 | 3,49 | 56,0 |
| Exemple 2 | os | 227,2 | 1,77 | 128,4 |
| Exemple 3 | os | 288,7 | 5,82 | 49,6 |

suite tableu I

| Composé | Voie d'administration | A<br>DL 50<br>mg/kg | B<br>DI 50<br>mg/kg | A/P |
|---|---|---|---|---|
| Exemple 4 | os | 202,5 | 5,69 | 35,6 |
| Exemple 5 | os | ~300 | 1,71 | ~175,5 |
| Exemple 6 | os | >600 | 4,75 | >126,3 |
| Exemple 7 | os | >800 | 10,44 | > 76,6 |
| Fenfluramine | i. p.<br>os | 102,1<br>230,9 | 1,58<br>2,51 | 64,6<br>92,0 |

De ce tableau, il ressort que les produits représentatifs de la présente invention montrent une bonne activité anorexigène avec une faible toxicité. Certains composés sont, sur le plan de l'indice thérapeutique, supérieurs au composé de référence.

Sur le plan biochimique, les 4-(3-trifluorométhylphényl)-1,2,3,6-tétrahydropyridines de la présente invention et leurs sels diffèrent de la fenfluramine et de ses dérivés dans leur mécanisme d'action. En fait, les composés de la présente invention ont une très bonne affinité pour les récepteurs post-synaptiques de la sérotonine avec des effets très faibles sur les mécanismes pré-synaptiques, tels que la libération de la sérotonine, sur lesquels, au contraire, la fenfluramine agit. Le mécanisme d'action des composés de la présente invention se traduit par une activité anorexigène remarquable et des effets secondaires réduits.

En particulier, les composés de la présente invention, in vivo, ne provoquent pas de déplétion de sérotonine au niveau central. Il y a donc une mineure possibilité que l'usage prolongé des composés de la présente invention provoque des effets secondaires au niveau central.

Le tableau II ci-dessous résume les taux cérébraux de sérotonine, en pourcentage par rapport aux contrôles, après administration intrapéritonéale ou orale de quatre composés représentatifs de la présente invention. La détermination des taux cérébraux, selon Curzon et Green (Br. J. Pharmacol. 1970, 39, 653), a été effectuée deux heures après l'administration. La fenfluramine a été utilisée comme produit de référence.

Tableau II

| Composé | Voie d'administration | dose mg/kg | taux cérébraux de sérotonine, % par rapport aux contrôles |
|---|---|---|---|
| Exemple 1 | i. p. | 1,625 | 93 ± 1 |
| | | 3,25 | 85 ± 1 |
| | | 7,5 | 98 ± 10 |
| Exemple 2 | os | 2,0 | 113 ± 5 |
| Exemple 3 | os | 2,5 | 132**) ± 7 |
| | | 5,0 | 134**) ± 2 |
| | | 10,0 | 126*) ± 5 |

suite tableaus II

| Composé | Voie d'administration | dose mg/kg | taux cérébraux de sérotonine, % par rapport aux contrôles |
|---|---|---|---|
| Exemple 4 | os | 5,69 | 119 ± 6 |
| Fenfluramine | i. p. | 7,5 | 53**) ± 4 |
| | os | 8,0 | 75**) ± 5 |

*) significatif P < 0,05
**) significatif P < 0,01

De ce tableau il ressort que les produits de la présente invention, à une dose supérieure à la DI 50 anorexigène, ne diminuent pas les taux cérébraux de sérotonine, tandis que la fenfluramine provoque une réduction significative de sérotonine cérébrale.

L'affinité des composés de la présente invention pour les récepteurs post-synaptiques de la sérotonine a été déterminée selon la méthode de Peroutka et Synder (Molec. Pharmacol. 1979, 16, 687) qui consiste à incuber des membranes de cortex de rat avec une concentration fixée de $^3$H-sérotonine en présence de différentes concentrations de produit. Le tableau III montre la concentration molaire de cinq produits représentatifs de la présente invention qui donne une inhibition de 50% de liaison spécifique au récepteur sérotononergique (CI 50) à savoir la mesure de la capacité du produit d'interagir avec la liaison de la$^3$H-sérotonine à son récepteur. La fenfluramine a été utilisée comme composé de référence.

Tableau III

| Composé | liaison $^3$H-sérotonine (2 mcM) CI 50 [M] |
|---|---|
| Exemple 1 | $1{,}2 \cdot 10^{-8}$ |
| Exemple 2 | $9{,}2 \cdot 10^{-9}$ |
| Exemple 3 | $5{,}4 \cdot 10^{-8}$ |
| Exemple 4 | $1{,}4 \cdot 10^{-8}$ |
| Exemple 6 | $1{,}5 \cdot 10^{-7}$ |
| Fenfluramine | $4{,}5 \cdot 10^{-6}$ |

De ce tableau il ressort que les composés de la présente invention ont une très bonne affinité pour le récepteur sérotoninergique post-synaptique alors que l'affinité du composé de référence pour le même récepteur est beaucoup plus faible.

Les composés de formule I ci-dessus sont peu toxiques et ils sont donc utiles comme médicaments.

Ainsi, selon un autre de ses aspects, la présente invention se réfère à des compositions pharmaceutiques renfermant, en tant qu'ingrédients actifs, des 4-(3-trifluorométhylphényl)-1,2,3,6-tétrahydropyridines de formule I ci-dessus, ainsi que leurs sels d'addition pharmaceutiquement acceptables.

Dans les compositions pharmaceutiques de la présente invention pour l'administration orale, sublinguale, souscutanée, intramusculaire, intraveineuse, transdermique ou rectale, les ingrédients actifs de formule I ci-dessus peuvent être administrés sous formes unitaires d'administration, en mélange avec des supports pharmaceutiques classiques, aux animaux et aux êtres humains pour le traitement de l'obésité. Les formes unitaires d'administration appropriées comprennent les formes par voie orale telles que les solutions ou suspensions orales, les formes d'administration sublinguale et buccale, les formes d'administration sous-cutanée, intramusculaire ou intraveineuse et les formes d'administration rectale.

Afin d'obtenir l'effet anorexigène désiré, la dose de principe actif peut varier entre 0,1 et 100 mg par kg de poids du corps et par jour.

Chaque dose unitaire peut contenir de 1 à 500 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore in peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à coté des 4-(3-trifluorométhylphényl)-1,2,3,6-tétrahydropyridines de la présente invention ou d'un de leurs sels pharmaceutiquement acceptables, d'autres principes actifs tels que, par exemple, des tranquillisants, des antidépresseurs, des hypolipémiants, des antidiabétiques ou d'autres médicaments qui peuvent être utiles dans le traitment de l'obésité.

Les exemples suivants illustrent l'invention sans toutefois la limiter.

Exemple 1

On chauffe au reflux pendant 24 h un mélange de 7,2 g de 4-(3-trifluorométhylphényl)-1,2,3,6-tétrahydropyridine, 6,4 g de triéthylamine, 5,63 g de chlorhydrate de 2-(2-chloroéthyl)pyridine dans 50 ml d'éthanol, puis on concentre le mélange réactionnel jusqu'à siccité. On reprend le résidu avec 300 ml d'éther diéthylique, on filtre, on lave la solution trois fois avec 50 ml d'eau et on la sèche sur du sulfate de sodium anhydre. Par acidification de la solution ainsi obtenue avec une solution de gaz chlorhydrique dans de l'isopropanol, on obtient un précipité (7 g) qui est cristallisé dans 150 ml d'éthanol. On obtient ainsi 5 g de chlorhydrate de 1-[2-(pyridyl)éthyl]-4-(3-trifluorométhylphényl)-1,2,3,6-tétrahydropyridine; p. f. 206—208°C.

Exemple 2

a) On agite 1 h à la température ambiante une suspension de 11,3 g de 3-pyridylacétate de p-nitrophényle et 10 g de 4-(3-trifluorométhylphényl)-1,2,3,6-tétrahydropyridine dans 150 ml d'éthanol, puis on concentre sous pression réduite. On dissout le résidu dans de l'éther diéthylique, on lave la solution à l'eau, puis avec de l'hydroxyde de sodium et encore à l'eau, puis on la sèche sur du sulfate de sodium anhydre et on la concentre. On obtient ainsi 8 g de 1-(3-pyridylacétyl)-4-(3-trifluorométhylphényl)-1,2,3,6-tétrahydropyridine, sous forme d'une huile qui est utilisée pour la réaction suivante.

b) A une suspension de 1,7 g d'hydrure de lithium et d'aluminium dans 30 ml d'éther diéthylique anhydre, on ajoute, par portions, une suspension de 8 g de 1-(3-pyridylacéthyl)-4-(3-trifluorométhylphényl)-1,2,3,6-tétrahydropyridine. La température est maintenue au dessous de 25°C. On agite le mélange réactionnel 1 h à la même température, puis on y ajoute lentement de l'eau. On filtre et on acidifie la solution organique avec une solution saturée de gaz chlorhydrique dans de l'isopropanol. On obtient un précipité (7 g) qui est cristallisé dans un mélange isopropanol : éthanol 14 : 1. On obtient ainsi 5 g de chlorhydrate de 1-[2-(3-pyridyl)éthyl]-4-(3-trifluorométhylphényl)-1,2,3,6-tétrahydropyridine; p.f. 217—219°C.

Exemple 3

On chauffee 6 h au reflux un mélange de 7,2 g de 4-(3-trifluorométhylphényl) 1,2,3,6-tétrahydropy-

ridine, 3,2 g de 4-vinylpyridine, 0,5 ml d'acide acétique et 50 ml d'éthanol à 95 %, puis on concentre jusqu'a siccité. On dissout le résidu dans 100 ml d'acétone et on filtre sur charbon. Par acidification de la solution avec une solution de gaz chlorhydrique de 1-[2-(4-pyridyl)éthyl]-4-(3-trifluorométhylphényl)-1,2,3,6-tétrahydropyridine qui, après cristallisation dans l'isopropanol, donnent 5,5 g de produit pur; p.f. 240—245°C.

Exemple 4

En opérant comme décrit dans l'Exemple 1, par réaction de 5,7 g de 4-(3-trifluorométhylphényl)-1,2,3,6-tétrahydropyridine avec 5,2 g de chlorhydrate de 2-(2-chloroéthyl)-6-méthylpyridine en présence de 7,5 ml de triéthylamine dans 60 ml d'éthanol, au reflux pendant 18 h, on obtient 3,5 g de chlorhydrate de 1-[2-(6-méthyl-2-pyridyl)éthyl]-4-(3-trifluorométhylphényl)-1,2,3,6-tétrahydropyridine; p.f. 207—210°C.

Exemple 5

En opérant comme décrit dans l'Exemple 3, par réaction de 5,7 g de 4-(3-trifluorométhylphényl)-1,2,3,6-tétrahydropyridine avec 2,5 g de 4-vinylpyridine 1-oxyde dans 25 ml d'alcool n-pentylique, au reflux pendant 24 h, on obtient 3,5 g de chlorhydrate de 4-[2-[4-(3-trifluorométhylphényl)-1,2,3,6-tétrahydropyrid-1-yl]éthyl]pyridine 1-oxyde; p.f. 158—160°C.

Exemple 6

a) A une solution de 7,5 g de 4-(3-trifluorométhylphényl)-1,2,3,6-tétrahydropyridine et 4,6 ml de triéthylamine dans 40 ml de chlorure de méthylène, on ajoute, goutte à goutte à 0°C, 6,75 g de chlorure de 1-naphtylacétyle dans 30 ml de chlorure de méthylène, puis on agite à 0°C pendant 2 h. On ajoute de l'eau, on sépare la phase organique, on la lave à l'eau plusieurs fois et on la concentre sous pression réduite. On obtient ainsi 11 g de produit brut qui, après cristallisation dans l'éthanol à 95 %, donnent 8 g de 1-(1-naphtylacétyl)-4-(3-trifluorométhylphényl)-1,2,3,6-tétrahydropyridine; p.f. 137—140°C.

b) A un mélange de 1,2 g d'hydrure de lithium et d'aluminium et 30 ml d'éther diéthylique anhydre on ajoute, goutte à goutte et à 25—30°C, une suspension de 7,5 g de 1-(1-naphtylacétyl)-4-(3-trifluorométhylphényl)-1,2,3,6-tétrahydropyridine dans 100 ml d'éther diéthylique anhydre, puis on agite 2 h à la température ambiante, on détruit avec de l'eau l'axcès d'hydrure de lithium et d'aluminium et on élimine la phase aqueuse. On lave à l'eau la phase éthérée, on la sèche sur du sulfate de sodium anhydre et on concentre sous pression réduite. On reprend le résidu avec de l'isopropanol, on traite la solution avec une solution de gaz chlorhydrique en isopropanol et on cristallise le précipité trois fois dans l'éthanol absolu. On obtient ainsi 3,5 g de chorhydrate de 1-[2-(1-naptyl)éthyl]-4-(3-trifluorométhylphényl)-1,2,3,6-tétrahydropyridine; p.f. 238—240°C.

Exemple 7

a) En opérant comme décrit dans l'Exemple 6 a), par réaction de 7 g de 4-(3-trifluorométhylphényl)-1,2,3,6-tétrahydropyridine et 6,15 g de chlorure de 2-naphtylacétyle en présence de 4,15 g de triéthylamine dans du chlorure de méthylène on obtient 9 g de 1-(2-naphtylacétyl)-4-(3-trifluorométhylphényl)-1,2,3,6-tétrahydropyridine; p.f. 125—130°C.

b) Suivant le mode opératoire décrit dans l'Exemple 6 b), par réducation du produit ci-dessus avec 1,5 g d'hydrure de lithium et d'aluminium, on obtient 5,5 g de chlorhydrate de 1-[2-(2-naphtyl)éthyl]-4-(3-trifluorométhylphényl)-1,2,3,6-tétrahydropyridine; p.f. 255—260°C.

Exemple 8

En opérant comme décrit dans l'Exemple 1, par réaction de la 4-(3-trifluorométhylphényl)-1,2,3,6-tétrahydropyridine avec la 4-(2-chloroéthyl)pyridine, la 4-(3-chloropropyl)-pyridine, la 4-(4-chlorobutyl)pyridine, la 2-(2-chloro-1-méthyléthyl)pyridine, la 2-(2-chloro-2-méthyléthyl)pyridine, la 4-(3-chloropropyl)pyridine 1-oxyde et, respectivement, la 4-(4-chlorobutyl)pyridine 1-oxyde, on obtient:

- le chlorhydrate de 1-[2-(4-pyridyl)éthyl]-4-(3-trifluorométhylphényl)-1,2,3,6-tétrahydropyridine;
- le chlorhydrate de 1-[3-(4-pyridyl)propyl]-4-(3-trifluorométhylphényl)-1,2,3,6-tétrahydropyridine;

- le chlorhydrate de 1-[4-(4-pyridyl)butyl]-4-(3-trifluorométhylphényl)-1,2,3,6-tétrahydropyridine;
- le chlorhydrate de 1-[2-(2-pyridyl)propyl]-4-(3-trifluorométhylphényl)-1,2,3,6-tétrahydropyridine;
- le chlorhydrate de 1-[1-méthyl-2-(2-pyridyl)éthyl]-4-(3-trifluorométhylphényl)-1,2,3,6-tétrahydropyridine;
- le chlorhydrate de 4-[3-[4-(3-trifluorométhylphényl)-1,2,3,6-tétrahydropyrid-1-yl]propyl]pyridine 1-oxyde et
- le chlorhydrate de 4-[4-[4-(3-trifluorométhylphényl)-1,2,3,6-tétrahydropyrid-1-yl]butyl]pyridine 1-oxyde.

De la même façon, en faisant réagir la 4-(3-trifluorométhylphényl)-1,2,3,6-tétrahydropyridine avec, respectivement, le 1-(2-chloroéthyl)naphtalène, le 1-(3-chloropropyl)-naphtalène, le 1-(4-chlorobutyl)naphtalène, le 2-(2-chloroethyl)naphtalène, le 2-(3-chloropropyl)naphtalène, le 2-(4-chlorobutyl)-naphtalène et le 2-(2-chloropropyl)naphtalène, on obtient:

- le chlorhydrate de 1-[2-(1-naphtyl)éthyl]-4-(3-trifluorométhylphényl)-1,2,3,6-tétrahydropyridine;
- le chlorhydrate de 1-[3-(1-naphtyl)propyl]-4-(3-trifluorométhylphényl)-1,2,3,6-tétrahydropyridine;
- le chlorhydrate de 1-[4-(1-naphtyl)butyl]-4-(3-trifluorométhylphényl)-1,2,3,6-tétrahydropyridine;
- le chlorhydrate de 1-[2-(2-naphtyl)éthyl]-4-(3-trifluorométhylphényl)-1,2,3,6-tétrahydropyridine;
- le chlorhydrate de 1-[3-(2-naphtyl)propyl]-4-(3-trifluorométhylphényl)-1,2,3,6-tétrahydropyridine;
- le chlorhydrate de 1-[4-(2-naphtyl)butyl]-4-(3-trifluorométhylphényl)-1,2,3,6-tétrahydropyridine et
- le chlorhydrate de 1-[1-méthyl-2-(2-naphtyl)éthyl]-4-(3-trifluorométhylphényl)-1,2,3,6-tétrahydropyridine.

## Exemple 9

a) Suivant le mode opératoire décrit dans l'Exemple 6 a), en faisant réagir la 4-(3-trifluorométhylphényl)-1,2,3,6-tétrahydropyridine avec, respectivement, le chlorure de l'acide 2-pyridylacétique, le chlorure de l'acide 3-(2-pyridyl)propionique, le chlorure de l'acide 1-naphtylacétique, le chlorure de l'acide 2-(1-naphtyl)propionique, le chlorure de l'acide 2-naphtylacétique et le chlorure de l'acide 2-(2-naphtyl)propionique dans du chlorure de méthylène, on obtient:

- la 1-(2-pyridylacétyl)-4-(3-trifluorométhylphényl)-1,2,3,6-tétrahydropyridine;
- la 1-[3-(2-pyridyl)propionyl]-4-(3-trifluorométhylphényl)-1,2,3,6-tétrahydropyridine;
- la 1-(1-naphtylacétyl)-4-(3-trifluorométhylphényl)-1,2,3,6-tétrahydropyridine;
- la 1-[3-(1-naphtyl)propionyl]-4-(3-trifluorométhylphényl)-1,2,3,6-tétrahydropyridine;
- la 1-(2-naphtylacétyl)-4-(3-trifluorométhylphényl)-1,2,3,6-tétrahydropyridine; et
- la 1-[3-(2-naphtyl)propionyl]-4-(3-trifluorométhylphényl)-1,2,3,6-tétrahydropyridine.

b) Suivant le mode opératoire décrit dans l'Exemple 6 b), par réduction des produits ainsi obtenus avec l'hydrure de lithium et d'aluminium, on obtient:

- le chlorhydrate de 1-[2-(2-pyridyl)éthyl]-4-(3-trifluorométhylphényl)-1,2,3,6-tétrahydropyridine;
- le chlorhydrate de 1-[3-(2-pyridyl)propyl]-4-(3-trifluorométhylphényl)-1,2,3,6-tétrahydropyridine;
- le chlorhydrate de 1-[2-(1-naphtyl)éthyl]-4-(3-trifluorométhylphényl)-1,2,3,6-tétrahydropyridine;
- le chlorhydrate de 1-[3-(1-naphtyl)propyl]-4-(3-trifluorométhylphényl)-1,2,3,6-tétrahydropyridine;
- le chlorhydrate de 1-[2-(2-naphtyl)éthyl]-4-(3-trifluorométhylphényl)-1,2,3,6-tétrahydropyridine et
- le chlorhydrate de 1-[3-(2-naphtyl)propyl]-4-(3-trifluorométhylphényl)-1,2,3,6-tétrahydropyridine.

## Exemple 10

On prépare des gélules à base d'un des composés des Exemples 1 à 9, ayant la composition suivante:

| | |
|---|---|
| principe actif | 15 mg |
| lactose | 120 mg |
| stéarate de magnésium | 5 mg |

en mélangeant intimement des charges des ingrédients ci-dessus et en versant le mélange dans des gélules de gélatine dure.

### Exemple 11

On prépares des comprimés à base d'un des composés des Exemple 1 à 9, ayant la composition suivante:

| | |
|---|---|
| principe actif | 20 mg |
| lactose | 100 mg |
| cellulose microcristalline | 30 mg |
| amidon de mais séché | 40 mg |
| stéarate de magnésium | 5 mg |

en broyant l'ingrédient actif jusqu'à une dimension particulaire de 0,4 mm en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg d'ingrédient actif.

### Exemple 12

En opérant comme décrit dans l'Exemple 11 ci-dessus, on prépare des comprimés ayant la composition suivante:

| | |
|---|---|
| principe actif | 50 mg |
| lactose | 95 mg |
| amidon de mais | 100 mg |
| talc | 4,5 mg |
| stéarate de magnésium | 0,5 mg |

### Exemple 13

On prépare des suppositoires à base d'un des composés des Exemples 1 à 9, ayant la composition suivante:

| | |
|---|---|
| principe actif | 50 mg |
| lactose | 250 mg |
| masse pour suppositoires q.s.p. | 1,7 g |

On mélange la substance active avec le lactose et on met le mélange uniformément en suspension dans la masse pour suppositoires fondue. On verse la suspension dans des moules refroidis pour former des suppositoires d'un poids de 1,7 g.

**Revendications pour les Etats contractants: BE, CH, DE, FR, GB, IT, LI, LU, NL, SE**

1. 4-(3-trifluorométhylphényl)-1,2,3,6-tétrahydropyridines de formule

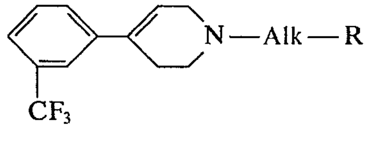

(I)

dans laquelle R représente un groupe pyridyle, un groupe pyridyle l-oxyde ou un groupe naphtyle, non substitués ou substitués par un groupe alkyle contenant de 1 à 4 atomes de carbone et Alk représente un groupe alkylène à chaine droite ou ramifiée contenant de 2 à 4 atomes de carbone, et leurs sels pharmaceutiquement acceptables.

2. La 4-[2-[4-(3-trifluorométhylphényl)-1,2,3,6-tétrahydropyrid-1-yl]éthyl]pyridine 1-oxyde et ses sels pharmaceutiquement acceptables.

3. Chlorhydrate de 4-[2-[4-(3-trifluorométhylphényl)-1,2,3,6-tétrahydropyrid-1-yl]éthyl]pyridine 1-oxyde.

4. Chlorhydrate de 1-[2-(2-pyridyl)éthyl]-4-(3-trifluorométhylphényl)-1,2,3,6-tétrahydropyridine.

5. Chlorhydrate de 1-[2-(3-pyridyl)éthyl]-4-(3-trifluorométhylphényl)-1,2,3,6-tétrahydropyridine.

6. Chlorhydrate de 1-[2-(4-pyridyl)éthyl]-4-(3-trifluorométhylphényl)-1,2,3,6-tétrahydropyridine.

7. Chlorhydrate de 1-[2-(6-méthyl-2-pyridyl)éthyl]-4-(3-trifluorométhylphényl)-1,2,3,6-tétrahydropyridine.

8. Chlorhydrate de 1-[2-(1-naphtyl)éthyl]-4-(3-trifluorométhylphényl)-1,2,3,6-tétrahydropyridine.

9. Chlorhydrate de 1-[2-(2-naphtyl)éthyl]-4-(3-trifluorométhylphényl)-1,2,3,6-tétrahydropyridine.

10. Procédé pour la préparation de 4-(3-trifluorométhylphényl)-1,2,3,6-tétrahydropyridines de formule I selon la revendication I, caractérisé en ce qu'on fait réagir, dans un solvant organique à une température de 20 à 200°C, la 4-(3-trifluorométhylphényl)-1,2,3,6-tétrahydropyridine de formule

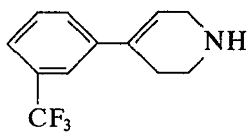

(II)

avec un composé de formule X—R, où R a la signification donnée ci-dessus et X représente un groupe chloro-, bromo- ou iodoalkyle ayant de 1 à 4 atomes de carbone ou un groupe électrophile, ou bien, lorsque R est autre que naphtyle éventuellement substitué, un groupe vinyle non substitué ou substitué par 1 ou 2 groupes méthyle ou par un groupe éthyle et l'on transforme éventuellement le produit ainsi obtenu dans ses sels pharmaceutiquement acceptables.

11. Procédé selon la revendication 10, caractérisé en ce que le solvant organique est un alcool aliphatique contenant 1 à 6 atomes de carbone.

12. Procédé pour la préparation de 4-(3-trifluorométhylphényl)-1,2,3,6-tétrahydropyridines de formule

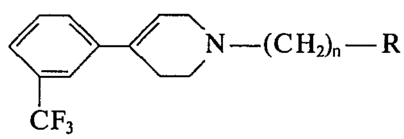

(III)

où R représente un groupe pyridyle, un groupe pyridyle 1-oxyde ou un groupe naphtyle, non substitués ou substitués par un groupe alkyle contenant de 1 à 4 atomes de carbone et n est 1, 2, 3 ou 4 et de leurs sels pharmaceutiquement acceptables, caractérisé en ce qu'on réduit un composé de formule

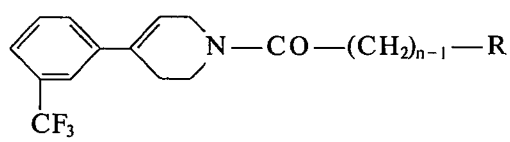

(IV)

dans laquelle R et n sont tels que définis ci-dessus, par un hydrure d'aluminium ou par un hydrure complexe de lithium et d'aluminium dans un solvant organique inerte à une témperature entre 0°C et la température d'ébullition du solvant employé et l'on transforme éventuellement le produit ainsi obtenu dans ses sels pharmaceutiquement acceptables.

13. Procédé selon la revendication 12, caractérisé en ce que comme agent de réduction on utilise l'hydrure de lithium et d'aluminium $LiAlH_4$.

14. Procédé selon une des revendications 12 et 13, caractérisé en ce que le composé IV est obtenu en faisant réagir la 4-(3-trifluorométhylphényl)-1,2,3,6-tétrahydropyridine de formule

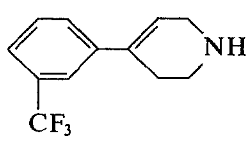

(II)

avec un dérivé fonctionnel d'un acide carboxylique de formule

$$R-(CH_2)_{n-1}-COOH \quad (V)$$

où R et n sont tels que définis dans la revendication 12, dans un solvant organique à une température comprise entre −10°C et la température d'ébullition du solvant employé.

15. Procéde selon la revendication 14, caractérisé en ce que comme dérivé fonctionnel de l'acide carboxylique V, on utilise le chlorure et l'on opére en présence d'un accepteur de protons.

16. Procéde selon une la revendication 15, caractérisé en ce que comme accepteurs de protons on utilise la triéthylamine.

17. Procédé selon la revendication 14, caractérisé en ce que comme dérivé fonctionnel de l'acide carboxylique V, on utilise l'ester de p-nitrophényle.

**Revendications pour l' Etat contractant: AT**

1. Procéde pour la préparation de 4-(3-trifluorométhylphényl)-1,2,3,6-tétrahydropyridines de formule

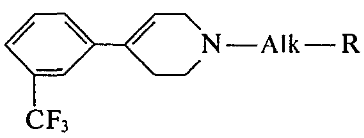

(I)

dans laquelle R représente un groupe pyridyle, un groupe pyridyle l-oxyde ou un groupe naphtyle, non substitués ou substitués par un groupe alkyle contenant de 1 à 4 atomes de carbone et Alk représente un groupe alkylène à chaine droite ou ramifiée contenant de 2 à 4 atomes de carbone, et de leurs sels pharmaceutiquement acceptables, caractérisé en ce qu'on fait réagir, dans un solvant organique à une température de 20 à 200°C, la 4-(3-trifluorométhylphényl)-1,2,3,6-tétrahydropyridine de formule

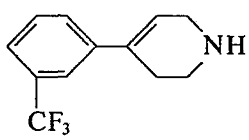

(II)

avec un composé de formule X—R, où R a la signification donnée ci-dessus et X représente un groupe chloro-, bromo- ou iodoalkyle ayant de 1 à 4 atomes de carbone ou un groupe électrophile, ou bien, lorsque R est autre que naphtyle éventuellement substitué, un groupe vinyle non substitué ou substitué par 1 ou 2 groupes méthyle ou par un groupe éthyle et l'on transforme éventuellement le produit ainsi obtenu dans ses sels pharmaceutiquement acceptables.

2. Procédé selon la revendication 1, caractérisé en ce que le solvant organique est un alcool aliphatique contenant 1 à 6 atomes de carbone.

3. Procédé pour la préparation de 4-(3-trifluorométhylphényl)-1,2,3,6-tétrahydropyridines de formule

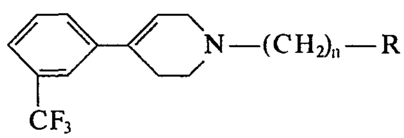

(III)

où R représente un groupe pyridyle, un groupe pyridyle 1-oxyde ou un groupe naphtyle, non substitués ou substitués par un groupe alkyle contenant de 1 à 4 atomes de carbone et n est 1, 2, 3 ou 4 et de leurs sels pharmaceutiquement acceptables, caractérisé en ce qu'on réduit un composé de formule

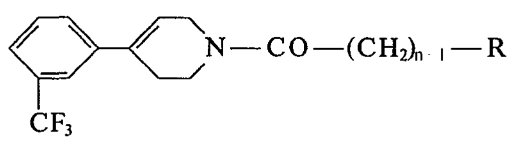

(IV)

dans laquelle R et n sont tels que définis ci-dessus, par un hydrure d'aluminium ou par un hydrure complexe de lithium et d'aluminium dans un solvant organique inerte à une témperature entre 0°C et la température d'ébullition du solvant employé et l'on transforme éventuellement le produit ainsi obtenu dans ses sels pharmaceutiquement acceptables.

4. Procédé selon la revendication 3, caractérisé en ce que comme agent de réduction on utilise l'hydrure de lithium et d'aluminium $LiAlH_4$.

5. Procédé selon une des revendications 3 et 4, caractérisé en ce que le composé IV est obtenu en faisant réagir la 4-(3-trifluorométhylphényl)-1,2,3,6-tétrahydropyridine de formule

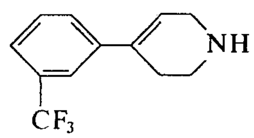

(II)

avec un dérivé fonctionnel d'un acide carboxylique de formule

$$R-(CH_2)_{n-1}-COOH \quad (V)$$

où R et n sont tels que définis dans la revendication 3, dans un solvant organique à une température comprise entre −10°C et la température d'ébullition du solvant employé.

6. Procéde selon la revendication 5, caractérisé en ce que comme dérivé fonctionnel de l'acide carboxylique V, on utilise le chlorure et l'on opére en présence d'un accepteur de protons.

7. Procéde selon une la revendication 6, caractérisé en ce que comme accepteurs de protons on utilise la triéthylamine.

8. Procédé selon la revendication 5, caractérisé en ce que comme dérivé fonctionnel de l'acide carboxylique V, on utilise l'ester de p-nitrophényle.

**Patentansprüche für die Vertragsstaaten: BE, CH, DE, FR, GB, IT, LI, LU, NL, SE**

1. 4-(3-Trifluormethylphenyl)-1,2,3,6-tetrahydropyridine der Formel

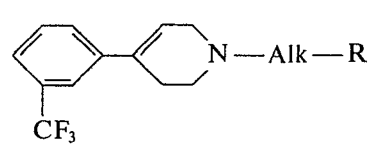

(I)

worin R eine nicht-substituierte oder eine durch eine 1 bis 4 Kohlenstoffatome enthaltende Alkylgruppe substituierte Pyridyl-, Pyridyl-1-oxid- oder Naphthylgruppe darstellt und Alk eine 2 bis 4 Kohlenstoffatome enthaltende Alkylengruppe mit gerader oder verzweigter Kette darstellt, und deren pharmazeutisch akzeptable Salze.

2. 4-[2-[4-(3-Trifluormethylphenyl)-1,2,3,6-tetrahydropyrid-1-yl]äthyl]pyridin-1-oxid und die pharmazeutisch akzeptablen Salze davon.

3. 4-[2-[4-(3-Trifluormethylphenyl)-1,2,3,6-tetrahydropyrid-1-yl]äthyl]pyridin-1-oxidhydrochlorid.

4. 1-[2-(2-Pyridyl)äthyl]-4-(3-trifluormethylphenyl)-1,2,3,6-tetrahydropyridinhydrochlorid.

5. 1-[2-(3-Pyridyl)äthyl]-4-(3-trifluormethylphenyl)-1,2,3,6-tetrahydropyridinhydrochlorid.

6. 1-[2-(4-Pyridyl)äthyl]-4-(3-trifluormethylphenyl)-1,2,3,6-tetrahydropyridinhydrochlorid.

7. 1-[2-(6-Methyl-2-pyridyl)äthyl]-4-(3-trifluormethylphenyl)-1,2,3,6-tetrahydropyridinhydrochlorid.

8. 1-[2-(1-Naphthyl)äthyl]-4-(3-trifluormethylphenyl)-1,2,3,6-tetrahydropyridinhydrochlorid.

9. 1-[2-(2-Naphthyl)äthyl]-4-(3-trifluormethylphenyl)-1,2,3,6-tetrahydropyridinhydrochlorid.

10. Verfahren zur Herstellung von 4-(3-Trifluormethylphenyl)-1,2,3,6-tetrahydropyridinen der Formel I gemäß Anspruch 1, dadurch gekennzeichnet, daß in einem organischen Lösungsmittel bei einer Temperatur von 20 bis 200°C das 4-(3-Trifluormethylphenyl)-1,2,3,6-tetrahydropyridin der Formel

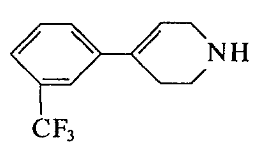

(II)

mit einer Verbindung der Formel X–R, worin R die oben angegebene Bedeutung hat und X eine Chlor-, Brom- oder Jodalkylgruppe mit 1 bis 4 Kohlenstoffatomen oder eine elektrophile Gruppe oder, falls R nicht Naphthyl, gegebenenfalls substituiert, ist, eine nicht-substituierte oder durch 1 oder 2 Methylgruppen oder durch eine Äthylgruppe substituierte Vinylgruppe darstellt, reagieren gelassen und das so erhaltene Produkt gegebenenfalls in seine pharmazeutisch akzeptablen Salze übergeführt wird.

11. Verfahren nach Anspruch 10, dadurch gekennzeichnet, daß das organische Lösungsmittel ein aliphatischer Alkohol mit 1 bis 6 Kohlenstoffatomen ist.

12. Verfahren zur Herstellung von 4-(3-Trifluormethylphenyl)-1,2,3,6-tetrahydropyridinen der Formel

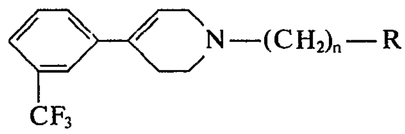

(III)

worin R eine nicht-substituierte oder eine durch eine 1 bis 4 Kohlenstoffatome enthaltende Alkylgruppe substituierte Pyridyl-, Pyridyl-1-oxid oder Naphthylgruppe darstellt und n 1, 2, 3 oder 4 ist, und den pharmazeutisch akzeptablen Salzen davon, dadurch gekennzeichnet, daß eine Verbindung der Formel

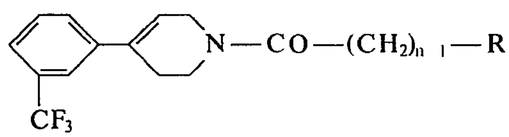

(IV)

worin R und n die oben angegebenen Bedeutung haben, mit einem Aluminiumhydrid oder einem Lithiumaluminiumhydridkomplex in einem inerten organischen Lösungsmittel bei einer Temperatur zwischen 0 °C und der Kochtemperatur des verwendeten Lösungsmittels reduziert und das so erhaltene Produkt gegebenenfalls in seine pharmazeutisch akzeptablen Salze übergeführt wird.

13. Verfahren nach Anspruch 12, dadurch gekennzeichnet, daß als Reduktionsmittel Lithiumaluminiumhydrid $LiAlH_4$ verwendet wird.

14. Verfahren nach einem der Ansprüche 12 und 13, dadurch gekennzeichnet, daß die Verbindung IV erhalten wird, indem das 4-(3-Trifluormethylphenyl)-1,2,3,6-tetrahydropyridin der Formel

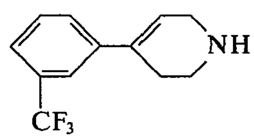

(II)

mit einem funktionellen Derivat einer Carbonsäure der Formel

$$R—(CH_2)_{n-1}—COOH \qquad (V)$$

worin R und n die in Anspruch 12 angegebenen Bedeutung haben, in einem organischen Lösungsmittel bei einer Temperatur zwischen —10°C und der Kochtemperatur des verwendeten Lösungsmittels reagieren gelassen wird.

15. Verfahren nach Anspruch 14, dadurch gekennzeichnet, daß als funktionelles Derivat der Carbonsäure V das Chlorid verwendet und in Gegenwart eines Protonenakzeptors gearbeitet wird.

16. Verfahren nach Anspruch 15, dadurch gekennzeichnet, daß als Protonenakzeptor Triäthylamin verwendet wird.

17. Verfahren nach Anspruch 14, dadurch gekennzeichnet, daß als funktionelles Derivat der Carbonsäure V p-Nitrophenylester verwendet wird.

**Patentansprüche für den Vertragsstaat: AT**

1. Verfahren zur Herstellung von 4-(3-Trifluormethylphenyl)-1,2,3,6-tetrahydropyridine der Formel

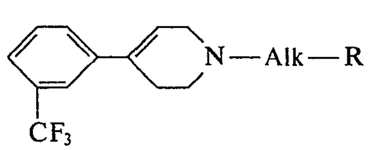

(I)

worin R eine nicht-substituierte oder eine durch eine 1 bis 4 Kohlenstoffatome enthaltende Alkylgruppe substituierte Pyridyl-, Pyridyl-1-oxid- oder Naphthylgruppe darstellt und Alk eine 2 bis 4 Kohlenstoffatome enthaltende Alkylengruppe mit gerader oder verzweigter Kette darstellt, und deren pharmazeutisch akzeptable Salze, dadurch gekennzeichnet, daß in einem organischen Lösungsmittel bei einer Temperatur von 20 bis 200°C das 4-(3-Trifluormethylphenyl)-1,2,3,6-tetrahydropyridin der Formel

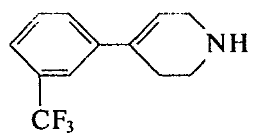

(II)

mit einer Verbindung der Formel X–R, worin R die oben angegebene Bedeutung hat und X eine Chlor-, Brom- oder Jodalkylgruppe mit 1 bis 4 Kohlenstoffatomen oder eine elektrophile Gruppe oder, falls R nicht Naphthyl, gegebenenfalls substituiert, ist, eine nicht-substituierte oder durch 1 oder 2 Methylgruppen oder durch eine Äthylgruppe substituierte Vinylgruppe darstellt, reagieren gelassen und das so erhaltene Produkt gegebenenfalls in seine pharmazeutisch akzeptablen Salze übergeführt wird.

2. Verfahren nach Anspruch 1, dadurch gekennzeichnet, daß das organische Lösungsmittel ein aliphatischer Alkohol mit 1 bis 6 Kohlenstoffatomen ist.

3. Verfahren zur Herstellung von 4-(3-Trifluormethylphenyl)-1,2,3,6-tetrahydropyridinen der Formel

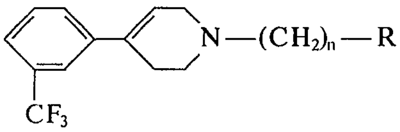

(III)

worin R eine nicht-substituierte oder eine durch eine 1 bis 4 Kohlenstoffatome enthaltende Alkylgruppe substituierte Pyridyl-, Pyridyl-1-oxid oder Naphthylgruppe darstellt und n 1, 2, 3 oder 4 ist, und den pharmazeutisch akzeptablen Salzen davon, dadurch gekennzeichnet, daß eine Verbindung der Formel

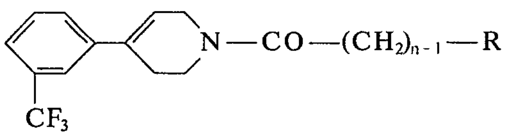

(IV)

worin R und n die oben angegebenen Bedeutungen haben, mit einem Aluminiumhydrid oder einem Lithiumaluminiumhydridkomplex in einem inerten organischen Lösungsmittel bei einer Temperatur zwischen 0°C und der Kochtemperatur des verwendeten Lösungsmittels reduziert und das so erhaltene Produkt gegebenenfalls in seine pharmazeutisch akzeptablen Salze übergeführt wird.

4. Verfahren nach Anspruch 3, dadurch gekennzeichnet, daß als Reduktionsmittel Lithiumaluminiumhydrid $LiAlH_4$ verwendet wird.

5. Verfahren nach einem der Ansprüche 3 und 4, dadurch gekennzeichnet, daß die Verbindung IV erhalten wird, indem das 4-(3-Trifluormethylphenyl)-1,2,3,6-tetrahydropyridin der Formel

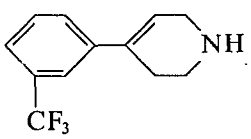

(II)

mit einem funktionellen Derivat einer Carbonsäure der Formel

$$R-(CH_2)_{n-1}-COOH \qquad (V)$$

worin R und n die in Anspruch 3 angegebenen Bedeutungen haben, in einem organischen Lösungsmittel bei einer Temperatur zwischen –10°C und der Kochtemperatur des verwendeten Lösungsmittels reagieren gelassen wird.

6. Verfahren nach Anspruch 5, dadurch gekennzeichnet, daß als funktionelles Derivat der Carbonsäure V das Chlorid verwendet und in Gegenwart eines Protonenakzeptors gearbeitet wird.

7. Verfahren nach Anspruch 6, dadurch gekennzeichnet, daß als Protonenakzeptor Triäthylamin verwendet wird.

8. Verfahren nach Anspruch 5, dadurch gekennzeichnet, daß als funktionelles Derivat der Carbonsäure V p-Nitrophenylester verwendet wird.

**Claims for the contracting States: BE, CH, DE, FR, GB, IT, LI, LU, NL, SE**

1. A 4-(3-trifluoromethylphenyl)-1,2,3,6-tetrahydropyridine of formula

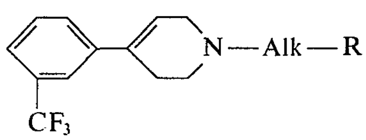

(I)

wherein R represents an unsubstitue or by an alkyl group of from 1 to 4 carbon atoms substituted pyridyl, pyridyl 1-oxide or naphthyl group an Alk represents a straight or branched chain alkylene group of from 2 to 4 carbon atoms, and the pharmaceutically acceptable salts thereof

2. The 4-[2-[4-(3-trifluoromethylphenyl)-1,2,3,6-tetrahydropyrid-1-yl]ethyl]pyridine 1-oxide and the pharmaceutically acceptable salts thereof.

3. The 4-[2-[4-(3-trifluoromethylphenyl)-1,2,3,6-tetrahydropyrid-1-yl]ethyl]pyridine 1-oxide hydrochloride.

4. The 1-[2-(2-pyridyl)ethyl]-4-(3-trifluoromethylphenyl)-1,2,3,6-tetrahydropyridine hydrochloride.

5. The 1-[2-(3-pyridyl)ethyl]-4-(3-trifluoromethylphenyl)-1,2,3,6-tetrahydropyridine hydrochloride.

6. The 1-[2-(4-pyridyl)ethyl]-4-(3-trifluoromethylphenyl)-1,2,3,6-tetrahydropyridine hydrochloride.

7. The 1-[2-(6-methyl-2-pyridyl)ethyl]-4-(3-trifluoromethylphenyl)-1,2,3,6-tetrahydropyridine hydrochloride.

8. The 1-[2-(1-naphthyl)ethyl]-4-(3-trifluoromethylphenyl)-1,2,3,6-tetrahydropyridine hydrochloride.

9. The 1-[2-(2-naphthyl)ethyl]-4-(3-trifluoromethylphenyl)-1,2,3,6-tetrahydropyridine hydrochloride.

10. A process for the preparation of 4-(3-trifluoromethylphenyl)-1,2,3,6-tetrahydropyridines of formula I according to claim I, characterized in that it comprises the steps of reacting the 4-(3-trifluoromethylphenyl)-1,2,3,6-tetrahydropyridine of formula

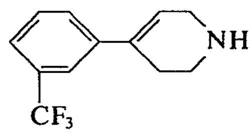

(II)

with a compound of formula X—R wherein R is as defined hereinabove and X represents a chloro-, bromo- or iodoalkyl group of from 1 to 4 carbon atoms or an electrophilic group or, when R is other than optionally substituted naphthyl, an unsubstituted or by 1 or 2 methyl groups or by an ethyl group substituted vinyl group in an organic solvent at a temperature of from 20 to 200°C and optionally converting the product thus obtained into the pharmaceutically acceptable salts thereof.

11. A process according to claim 10, characterized in that the organic solvent is an aliphatic alcohol of from 1 to 6 carbon atoms.

12. A process for the preparation of 4-(3-trifluoromethylphenyl)-1,2,3,6-tetrahydropyridines of formula

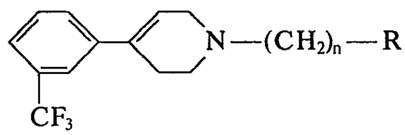

(III)

wherein R represents an unsubstituted or by an alkyl group of from 1 to 4 carbon atoms substituted pyridyl, pyridyl 1-oxide or naphthyl group and n represents one of the integers 1, 2, 3 or 4 and of the pharmaceutically acceptable salts thereof, characterized in that it comprises the step of reducing a compound of formula

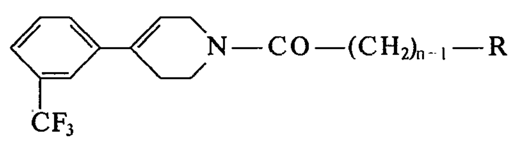

(IV)

wherein R and n are as defined above, by an aluminium hydride or by a lithium and aluminium hydride complex in an inert organic solvent at a temperature of from 0°C to the boiling temperature of the solvent employed and optionally converting the product thus obtained into pharmaceutically acceptable salts thereof.

13. A process according to claim 12, characterized in that the reducing agent is lithium and aluminium hydride $LiAlH_4$.

14. A process according to any one of claims 12 and 13, characterized in that the compound IV is obtained by reacting a 4-(3-trifluoromethylphenyl)-1,2,3,6-tetrahydropyridine of formula

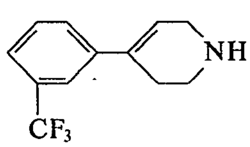

(II)

with a functional derivate of a carboxylic acid of formula

$$R—(CH_2)_{n-1}—COOH \qquad (V)$$

wherein R and n are as defined in claim 12, in an organic solvent at a temperature of from −10°C to the boiling temperature of the solvent employed.

15. A process according to claim 14, characterized in that the functional derivative of the carboxylic acid V is the chloride and the reaction is carried out in the presence of a proton acceptor.

16. A process according to claim 15, characterized in that the proton acceptor is triethylamine.

17. A process according to claim 14, characterized in that the functional derivative of the carboxylic acid V is the p-nitrophenyl ester.

**Claims for the contracting State: AT**

1. A process for the preparation of 4-(3-trifluoromethylphenyl)-1,2,3,6-tetrahydropyridines of formula

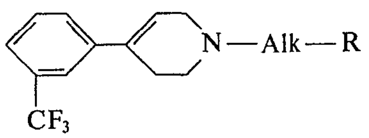

(I)

wherein R represents an unsubstitued or by an alkyl group of from 1 to 4 carbon atoms substituted pyridyl, pyridyl 1-oxide or naphthyl group an Alk represents a straight or branched chain alkylene group of from 2 to 4 carbon atoms, and the pharmaceutically acceptable salts thereof, characterized in that it comprises the steps of reacting the 4-(3-trifluoromethylphenyl)-1,2,3,6-tetrahydropyridine of formula

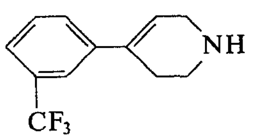

(II)

with a compound of formula X–R wherein R is as defined hereinabove and X represents a chloro-, bromo- or iodoalkyl group of from 1 to 4 carbon atoms or an electrophilic group or, when R is other than optionally substituted naphthyl, an unsubstituted or by 1 or 2 methyl groups or by an ethyl group substituted vinyl group in an organic solvent at a temperature of from 20 to 200°C and optionally converting the product thus obtained into the pharmaceutically acceptable salts thereof.

2. A process according to claim 1, characterized in that the organic solvent is an aliphatic alcohol of from 1 to 6 carbon atoms.

3. A process for the preparation of 4-(3-trifluoromethylphenyl)-1,2,3,6-tetrahydropyridines of formula

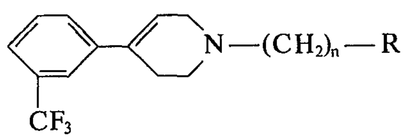

(III)

wherein R represents an unsubstituted or by an alkyl group of from 1 to 4 carbon atoms substituted pyridyl, pyridyl 1-oxide or naphthyl group and n represents one of the integers 1, 2, 3 or 4 and of the pharmaceutically acceptable salts thereof, characterized in that it comprises the step of reducing a compound of formula

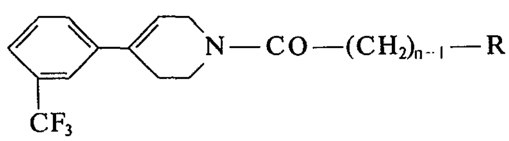

(IV)

wherein R and n are as defined above, by an aluminium hydride or by a lithium and aluminium hydride complex in an inert organic solvent at a temperature of from 0°C to the boiling temperature of the solvent employed and optionally converting the product thus obtained into the pharmaceutically acceptable salts thereof.

4. A process according to claim 3, characterized in that the reducing agent is lithium and aluminium hydride $LiAlH_4$.

5. A process according to any one of claims 3 and 4, characterized in that the compound IV is obtained by reacting a 4-(3-trifluoromethylphenyl)-1,2,3,6-tetrahydropyridine of formula

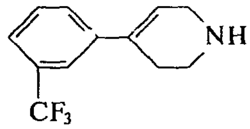 (II)

with a functional derivate of a carboxylic acid of formula

$$R—(CH_2)_{n-1}—COOH \qquad (V)$$

wherein R and n are as defined in claim 3, in an organic solvent at a temperature of from —10°C to the boiling temperature of the solvent employed.

6. A process according to claim 5, characterized in that the functional derivative of the carboxylic acid V is the chloride and the reaction is carried out in the presence of a proton acceptor.

7. A process according to claim 6, characterized in that the proton acceptor is triethylamine.

8. A process according to claim 5, characterized in that the functional derivative of the carboxylic acid V is the p-nitrophenyl ester.